# EUROPEAN PATENT APPLICATION

(11) **EP 4 527 938 A1**
(43) Date of publication of application: **26.03.2025**
(21) Application number: 23198962.5
(22) Date of filing: 22.09.2023
(51) Int. Cl.: C12P 21/02

(54) **METHOD FOR CELL-FREE POLYPEPTIDE SYNTHESIS**

(71) Applicant: LenioBio GmbH, 40231 Düsseldorf (DE)
(72) Inventor: BÜCHS, Jochen, 52062 Aachen (DE); STRAATEN, Sarah, 52078 Aachen (DE); ROENTGEN, Robin, 52062 Aachen (DE); FLASKAMP, Yannick, 52076 Aachen (DE)
(74) Representative: Maikowski & Ninnemann Patentanwälte Partnerschaft mbB

(57) **Abstract**

The present invention relates to a method for cell-free polypeptide synthesis, comprising translating mRNA in a cell-free reaction mixture under agitation of the cell-free reaction mixture. According to an aspect of the invention, the agitation is performed with a power input into the cell-free reaction mixture of at least 0.5 kW/m³. Furthermore, the cell-free reaction mixture comprises a polyether in a concentration lying in a range from 0.001 % (w/v) to 2 % (w/v), wherein the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol, as shown for poloxamer 188 (Pluronic F-68), Pluronic F-88, F-77, F-38, F-108, L-64, L-35 and L-31.

## Description

The present invention relates to a method for cell-free polypeptide synthesis according to the preamble of claim 1, to the use of a specific polyether in cell-free polypeptide synthesis according to claim 10, and to a cell-free reaction mixture for cell-free polypeptide synthesis according to the preambles of claims 11 and 15.

Cell-free polypeptide synthesis is well known from prior art. E.g., WO 2018/148530 A1 describes a system for cell-free protein synthesis. However, the presently available systems and methods for cell-free polypeptide synthesis are often limited with respect to the volume of the reaction mixture and thus with respect to the total amount of polypeptide that can be synthesized in a single reaction batch. A scale-up of the reaction volume typically does not lead to a linear increase of yield since the reaction mixture may require an amount of oxygen that is sufficient for effective polypeptide synthesis and that depends on various factors such as the nature and geometry of the reaction vessel, reaction volume, the concentration of the reactants, the surface of the reaction mixture, the power input into the reaction mixture, and the oxygen transfer rate into the reaction mixture. The power input and the oxygen transfer rate therefrom inherently resulting are the dominant parameters for the scale-up.

By agitating the reaction mixture, required oxygen can be introduced into the reaction mixture. However, the inventors observed that the polypeptide yield significantly decreases with increasing power input (due to agitation) into the reaction mixture. Obviously, the disadvantages for the polypeptide synthesis system due to the introduced kinetic energy outweighs the advantages due to the introduced additional oxygen.

WO 2005/098048 A1 describes a method in which antifoam agents are added to a cell-free protein synthesis system implemented in a bubble-column reactor in order to avoid foaming-out of the reaction mixture out of the bubble-column reactor and to enhance the yield of protein expression. This yield enhancement was shown for some specific antifoam agents in a concentration of from 0.0007 % to 0.007 % by weight in cell-free reaction mixtures having a volume of more than 15 µl.

The present inventors tried various antifoam agents belonging to the chemical groups disclosed in this international patent application, however without success for yield enhancement at high power input into the reaction mixture.

EP 1 507 003 A1 discloses further detergents that act as antifoaming agents and that can be used in cell-free protein synthesis. It should be noted that there is significant overlap between the substances disclosed in WO 2005/098048 A1 and EP 1 507 003 A1.

Tharmalingam et al. (2008) describes that Pluronic F-68 enhances cell yield in agitated cultures of serum-free Chinese hamster ovary (CHO) cells and reduce cell adhesion in stationary cultures. The authors concluded that the observed effects of Pluronic F-68 on these cultures are likely induced by a protective layer formed by Pluronic F-68 on the cell membrane. This layer is also denoted as artificial wall that appears to protect cells and even to repair damaged cells. The authors noted that the membrane surface coating of Pluronic F-68 serves to reduce the overall hydrophobicity of the cells. This reduced hydrophobicity results in an enhanced robustness, reduced surface attachment, and even a limited repair of damaged cells.

Gigout et al. (2008) investigated the protective mechanism of Pluronic F-68 in detail and demonstrated that Pluronic F-68 enters CHO cells and chondrocytes and possibly accumulates in the endocytic pathway. The results presented in this publication show that the uptake of Pluronic F-68 by the cells can severely affect Pluronic F-68 concentration in the culture medium and thus the shear protection effect.

It is an object of the present invention to provide a method for cell-free polypeptide synthesis that allows a high yield of synthesized polypeptide even in case of larger reaction mixture volumes.

This object is achieved with a method for cell-free polypeptide synthesis having the claim elements of claim 1. This method comprises the step of translating mRNA in a cell-free reaction mixture under agitation of the cell-free reaction mixture.

According to an aspect of the present invention, the agitation is performed with a power input into the cell-free reaction mixture of at least 0.5 kW/m³. In addition, the cell-free reaction mixture comprises a polyether in a concentration lying in a range of from 0.001 % (weight per volume; w/v) to 2 % (w/v). In this context, the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol.

Surprisingly, the inventors found out that the addition of such a specific polyether in the indicated concentration range serves for significant increase in polypeptide yield with respect to a reaction mixture not containing such a polyether at power inputs of at least 0.5 kW/m³ (but optionally containing other antifoaming agents such as various poloxamers not fulfilling the above-mentioned physicochemical properties). Depending on the chosen concentration within the precedingly mentioned concentration range, yields could be obtained lying in a range between 75 % and 100 % of the yield in a reference reaction mixture in which the cell-free polypeptide synthesis is performed with sufficient oxygen availability (i.e., a dissolved oxygen concentration above 0 %) at only slight agitation and low power input of approximately 0.2 kW/m³. This result was very surprising since many antifoam agents that have been disclosed in prior art did not show any comparable effect but did simply not enhance the yield of polypeptide synthesis regardless of the antifoam concentration.

Thus, the inventors were able to make a specific selection of a defined group of polyethers in a specific concentration range out of a plurality of known detergents and other antifoaming agents that are described in prior art for supplementing cell-free polypeptide synthesis reaction mixtures for yield enhancement. However, those other substances did not show a relevant effect when the reaction mixture is agitated with a comparable high power input (i.e., a power input of at least 0.5 kW/m³) required in particular at larger scale to obtain an oxygen input into the reaction mixture that is sufficient for an efficient protein synthesis.

The oxygen input into a system can be quantified by the oxygen transfer rate (OTR). For an efficient cell-free protein synthesis, a minimal OTR of 15 mmol/l/h is preferred. In an embodiment, the minimal OTR lies in a range of from 5 to 100 mmol/l/h, in particular from 10 to 95 mmol/l/h, in particular from 15 to 90 mmol/l/h, in particular from 20 to 85 mmol/l/h, in particular from 25 to 80 mmol/l/h, in particular from 30 to 70 mmol/l/h, in particular from 35 to 60 mmol/l/h, in particular from 40 to 50 mmol/l/h. Typically, the OTR increases linearly with increasing shaking frequency of a container comprising the cell-free reaction mixture, wherein the power input into the reaction mixture increases at the same time exponentially. In an embodiment, any of the beforementioned values of the OTR is combined with any of the specific embodiments of the power input disclosed below.

The directed choice of these polyethers for achieving a yield enhancement in a cell-free protein synthesis system that is agitated under high power input is particularly surprising since the scientific literature on specific examples of these polyethers would not make a person skilled in the art think that the effect observed in cell culture could be transferred to a cell-free protein synthesis system. Specifically, the publication of Gigout et al. (2008) relating to Pluronic F-68, which is also known as poloxamer 188, clearly describes that all cell-protective effects that are mediated by this substance and that counteracts effects arising from shear stress because of agitation of and gas sparging in a reaction mixture can be observed since the Pluronic F-68 enters into the cell to be protected. It was completely surprising to see that polyethers like poloxamer 188 can also protect the substances and/or cellular organelles contained in a cell-free protein synthesis system although - by definition - no cells are present in such a system. Thus, the yield-enhancing and protective effect of polyethers like poloxamer 188 must have a different physicochemical reason than the protective effect observed in case of cell cultures. While the structural details of this effect have not yet been fully understood, the effect was clearly seen for a plurality of polyethers fulfilling the criteria as defined in claim 1.

In an embodiment, the polyether fulfils one of the criteria explained in the following paragraphs.

According to the first criterion, the weight percentage of the hydrophilic units lies in a range of from 80 % to 100 % and the molecular weight of the polyether lies in a range of from 200 g/mol to 20000 g/mol. This criterion is applied since it turned out that highly hydrophilic substances of fully hydrophilic substances (such as polyethylene glycols) can be very well used for yield enhancement of cell-free protein synthesis under high power input irrespective of their concrete molecular weight. Thus, highly or fully hydrophilic substances are well suited as yield-enhancing additive for a cell-free protein synthesis system even if they have a particularly high molecular weight (i.e., 50,000 g/mol or more) or a particularly low molecular weight (i.e., 1000 g/mol or less).

According to the second criterion, the weight percentage of the hydrophilic units lies in a range of from 30 % to 79.9 % (i.e., just up to but not including 80 %) and the molecular weight of the hydrophobic units lies in a range of from 200 g/mol to 3500 g/mol. This criterion can be applied since it turned out that polyethers with a pronounced but not extremely high hydrophilicity (i.e., the weight percentage of the hydrophilic units ranging from 30 % to 79.9 %) show the desired yield-enhancing effect upon high power input into the cell-free protein synthesis in particular if their molecular weight is not too high. If the molecular weight of the hydrophobic units significantly exceeds 3500 g per mole, only a limited yield-enhancing effect could be shown for polyethers having a weight percentage of hydrophilic units from 30 % up to below 80 %.

According to the third criterion, the weight percentage of the hydrophilic units lies in a range of from 10 % to 29.9 % (i.e., just up to but not including 30 %) and the molecular weight of the hydrophobic units lies in a range of from 200 g/mol to 1500 g/mol. This criterion can be applied since it turned out that polyethers with a lower hydrophilicity (i.e., the weight percentage of the hydrophilic units ranging from 10 % to 29.9 %) show the desired yield-enhancing effect upon high power input into the cell-free protein synthesis system in particular if their molecular weight is even smaller than in case of polyethers having a pronounced but not extremely high hydrophilicity (i.e., the weight percentage of the hydrophilic units ranging from 30 % to 79.9 %). If the molecular weight of the hydrophobic units significantly exceeds 1500 g per mole, only a limited yield-enhancing effect could be shown for polyethers having a weight percentage of hydrophilic units from 10 % up to below 30 %.

In an embodiment, the polyether comprises at least one hydrophilic block of hydrophilic units and at least one hydrophobic block of hydrophobic units. Expressed in other words, the polyether is a block-copolymer in this embodiment. Such a block-copolymer comprising at least one hydrophilic block and at least one hydrophobic block typically has amphiphilic properties and can thus act as detergent. These amphiphilic properties probably assist in the yield-enhancing effect of the polyether.

In an embodiment, the polyether comprises one central hydrophobic block and two lateral hydrophilic blocks. In this context, each hydrophilic block is covalently bound to a different terminus of the hydrophobic block. Thus, the polyether has, in this embodiment, an appearance of a hydrophilic block followed by a hydrophobic block followed again by a hydrophilic block. Such a polyether can also be denoted as poloxamer if the hydrophilic units are polyethylene oxide) units and if the hydrophobic units are polypropylene oxide) units. Such poloxamers turned out to be particularly appropriate for achieving the yield-enhancing effect on cell-free protein synthesis system that is operated under high power input.

In an embodiment, the chemical structure of the polyether complies with the following general formula (I):

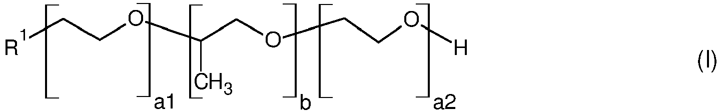

In this context, residue R¹ denotes -SH, -CH₃, -CH₂CH₃, -NH₂, -OH, -OCH₃, -NH₃⁺, -OPO₃⁻ (CH₂)₂N+(CH₃)₃, -O-C₁₄H₂₁, -O-C₆H₄-C(CH₃)₂-CH₂-C(CH₃)₂-CH₃, or -OSO₃⁻. In an embodiment, R¹ denotes -OH.

Thus, if b is bigger than zero, the polyether comprises a central hydrophobic chain of b monomer units of polypropylene oxide) flanked by two lateral chains of a monomer units of poly(ethylene oxide), wherein a = a1 + a2. In this embodiment, the polyether is a poloxamer. If b is zero, the polyether comprises only a1 + a2 units of polyethylene oxide). In such an embodiment, the polyether is a polyethylene glycol.

In an embodiment, the polyether is a poloxamer that is chosen from the group consisting of poloxamer 188, Pluronic F-88, Pluronic F-77, Pluronic F-38, Pluronic F-108, Pluronic L-64, Pluronic L-35, and Pluronic L-31. These substances turned out to be particularly appropriate for enhancing the yield of cell-free protein synthesis upon performing such synthesis with a high power input of at least 0.5 kW/m³.

In an embodiment, the polyether is a 4-(1,1,3,3-tetramethylbutyl)phenyl-polyethylene glycol, in particular a 4-(1,1,3,3-tetramethylbutyl)phenyl-polyethylene glycol marketed under the name Triton, in particular Triton X-405 (CAS Number: 9036-19-5; PubChem Substance ID: 329830805).

In an embodiment, the index a lies in a range of from 80 to 300, in particular from 85 to 295, in particular from 90 to 290, in particular from 95 to 285, in particular from 100 to 280, in particular from 105 to 275, in particular from 110 to 270, in particular from 115 to 265, in particular from 120 to 260, in particular from 125 to 255, in particular from 130 to 250, in particular from 135 to 245, in particular from 140 to 240, in particular from 145 to 235, in particular from 150 to 230, in particular from 155 to 225, in particular from 160 to 220, in particular from 165 to 215, in particular from 170 to 210, in particular from 175 to 205, in particular from 180 to 200, in particular from 185 to 195. This embodiment is in particular appropriate if the weight percentage of the hydrophilic units lies in a range of from 75 to 85 %, in particular around (±1 %) or at 80 %.

In an embodiment, the index a lies in a range of from 50 to 210, in particular from 55 to 205, in particular from 60 to 200, in particular from 65 to 195, in particular from 70 to 190, in particular from 75 to 185, in particular from 80 to 180, in particular from 85 to 175, in particular from 90 to 170, in particular from 95 to 165, in particular from 100 to 160, in particular from 105 to 155, in particular from 110 to 150, in particular from 115 to 145, in particular from 120 to 140, in particular from 125 to 135. This embodiment is in particular appropriate if the weight percentage of the hydrophilic units lies in a range of from 65 to 75 %, in particular around (± 1 %) or at 70 %.

In an embodiment, the index a lies in a range of from 5 to 100, in particular from 10 to 95, in particular from 15 to 90, in particular from 20 to 85, in particular from 25 to 80, in particular from 30 to 75, in particular from 35 to 70, in particular from 40 to 65, in particular from 45 to 60, in particular from 50 to 55. This embodiment is in particular appropriate if the weight percentage of the hydrophilic units lies in a range of from 45 to 55 %, in particular around (± 1 %) or at 50 %, or in a range of from 35 to 45 %, in particular around (± 1 %) or at 40 %.

In an embodiment, the index a lies in a range of from 2 to 35, in particular from 3 to 33, in particular from 4 to 31, in particular from 5 to 29, in particular from 6 to 27, in particular from 7 to 25, in particular from 8 to 23, in particular from 9 to 21, in particular from 10 to 19, in particular from 11 to 17, in particular from 12 to 15. This embodiment is in particular appropriate if the weight percentage of the hydrophilic units lies in a range of from 25 to 35 %, in particular around (± 1 %) or at 30 %.

In an embodiment, the index a lies in a range of from 2 to 15, in particular from 3 to 14, in particular from 4 to 13, in particular from 5 to 12, in particular from 6 to 11, in particular from 7 to 10, in particular from 8 to 9. This embodiment is in particular appropriate if the weight percentage of the hydrophilic units lies in a range of from 15 to 25 %, in particular around (± 1 %) or at 20 %.

In an embodiment, the index a lies in a range of from 1 to 3, in particular from 2 to 3. This embodiment is in particular appropriate if the weight percentage of the hydrophilic units lies in a range of from 5 to 15 %, in particular around (± 1 %) or at 10 %.

In an embodiment, the indices a and b have the meanings listed in Tables 1 to 7:

**Table 1: Particularly appropriate combinations of a and b for the indicated weight percentage range of the hydrophilic units.**

| Weight percentage of the hydrophilic units | a | b |
|---|---|---|
| 75 to 85 %, in particular 80 % | 80 to 300 | 15 to 60 |
| | 85 to 295 | 16 to 59 |
| | 90 to 290 | 17 to 58 |
| | 95 to 285 | 18 to 57 |
| | 100 to 280 | 19 to 56 |
| | 105 to 275 | 20 to 55 |
| | 110 to 270 | 21 to 54 |
| | 115 to 265 | 22 to 53 |
| | 120 to 260 | 23 to 52 |
| | 125 to 255 | 24 to 51 |
| | 130 to 250 | 25 to 50 |
| | 135 to 245 | 26 to 49 |
| | 140 to 240 | 27 to 48 |
| | 145 to 235 | 28 to 47 |
| | 150 to 230 | 29 to 46 |
| | 155 to 225 | 30 to 45 |
| | 160 to 220 | 31 to 44 |
| | 165 to 215 | 32 to 43 |
| | 170 to 210 | 33 to 42 |
| | 175 to 205 | 34 to 41 |
| | 180 to 200 | 35 to 40 |
| | 185 to 195 | 36 to 39 |

**Table 2: Particularly appropriate combinations of a and b for the indicated weight percentage range of the hydrophilic units.**

| Weight percentage of the hydrophilic units | a | b |
|---|---|---|
| 65 to 75 %, in particular 70 % | 50 to 210 | 15 to 60 |
| | 55 to 205 | 16 to 59 |
| | 60 to 200 | 17 to 58 |
| | 65 to 195 | 18 to 57 |
| | 70 to 190 | 19 to 56 |
| | 75 to 185 | 20 to 55 |
| | 80 to 180 | 21 to 54 |
| | 85 to 175 | 22 to 53 |
| | 90 to 170 | 23 to 52 |
| | 95 to 165 | 24 to 51 |
| | 100 to 160 | 25 to 50 |
| | 105 to 155 | 26 to 49 |
| | 110 to 150 | 27 to 48 |
| | 115 to 145 | 28 to 47 |
| | 120 to 140 | 29 to 46 |
| | 125 to 135 | 30 to 45 |

**Table 3: Particularly appropriate combinations of a and b for the indicated weight percentage range of the hydrophilic units.**

| Weight percentage of the hydrophilic units | a | b |
|---|---|---|
| 45 to 55 %, in particular 50 % | 5 to 100 | 5 to 100 |
| | 10 to 95 | 10 to 95 |
| | 15 to 90 | 15 to 90 |
| | 20 to 85 | 20 to 85 |
| | 25 to 80 | 25 to 80 |
| | 30 to 75 | 30 to 75 |
| | 35 to 70 | 35 to 70 |
| | 40 to 65 | 40 to 65 |
| | 45 to 60 | 45 to 60 |
| | 50 to 55 | 50 to 55 |

**Table 4: Particularly appropriate combinations of a and b for the indicated weight percentage range of the hydrophilic units.**

| Weight percentage of the hydrophilic units | a | b |
|---|---|---|
| 35 to 45 %, in particular 40 % | 5 to 100 | 5 to 100 |
| | 10 to 95 | 10 to 95 |
| | 15 to 90 | 15 to 90 |
| | 20 to 85 | 20 to 85 |
| | 25 to 80 | 25 to 80 |
| | 30 to 75 | 30 to 75 |
| | 35 to 70 | 35 to 70 |
| | 40 to 65 | 40 to 65 |
| | 45 to 60 | 45 to 60 |
| | 50 to 55 | 50 to 55 |

**Table 5: Particularly appropriate combinations of a and b for the indicated weight percentage range of the hydrophilic units.**

| Weight percentage of the hydrophilic units | a | b |
|---|---|---|
| 25 to 35 %, in particular 30 % | 2 to 35 | 5 to 65 |
| | 3 to 33 | 8 to 62 |
| | 4 to 31 | 11 to 59 |
| | 5 to 29 | 14 to 56 |
| | 6 to 27 | 17 to 53 |
| | 7 to 25 | 20 to 50 |
| | 8 to 23 | 23 to 47 |
| | 9 to 21 | 26 to 44 |
| | 10 to 19 | 29 to 41 |
| | 11 to 17 | 30 to 40 |
| | 12 to 15 | 33 to 37 |

**Table 6: Particularly appropriate combinations of a and b for the indicated weight percentage range of the hydrophilic units.**

| Weight percentage of the hydrophilic units | a | b |
|---|---|---|
| 15 to 25 %, in particular 20 % | 2 to 15 | 5 to 50 |
| | 3 to 14 | 9 to 46 |
| | 4 to 13 | 13 to 42 |
| | 5 to 12 | 17 to 38 |
| | 6 to 11 | 21 to 34 |
| | 7 to 10 | 25 to 30 |
| | 8 to 9 | 26 to 29 |

**Table 7: Particularly appropriate combinations of a and b for the indicated weight percentage range of the hydrophilic units.**

| Weight percentage of the hydrophilic units | a | b |
|---|---|---|
| 5 to 15 %, in particular 10 % | 1 to 3 | 8 to 25 |
| | 2 to 3 | 15 to 25 |

In an embodiment, the index a lies in a range of from 135 to 175, in particular of from 140 to 170, in particular of from 145 to 165, in particular of from 150 to 160, in particular of from 155 to 158. In an embodiment, the index b lies in a range of from 20 to 40, in particular of from 23 to 36, in particular of from 25 to 35, in particular of from 27 to 33, in particular of from 29 to 31. This embodiment is in particular appropriate if the weight percentage of the hydrophilic units lies in a range of from 75 to 85 %, in particular around (± 1 %) or at 80 %. In this context, the combinations of a and b listed in Table 8 are particularly appropriate:

**Table 8: Particularly appropriate combinations of a and b for the indicated weight percentage range of the hydrophilic units.**

| Weight percentage of the hydrophilic units | a | b |
|---|---|---|
| 75 to 85 %, in particular 80 % | 135 to 175 | 20 to 40 |
| | 140 to 170 | 23 to 36 |
| | 145 to 165 | 25 to 35 |
| | 150 to 160 | 27 to 33 |
| | 155 to 158 | 29 to 31 |

In an embodiment, the polyether has a mean molecular weight, determined by size exclusion chromatography, lying in a range of from 250 g/mol to 20000 g/mol, in particular from 300 g/mol to 19000 g/mol, in particular from 400 g/mol to 18000 g/mol, in particular from 500 g/mol to 17000 g/mol, in particular from 600 g/mol to 16000 g/mol, in particular from 700 g/mol to 15000 g/mol, in particular from 800 g/mol to 14000 g/mol, in particular from 900 g/mol to 13000 g/mol, in particular from 1000 g/mol to 12000 g/mol, in particular from 1100 g/mol to 11000 g/mol, in particular from 1200 g/mol to 10000 g/mol, in particular from 1300 g/mol to 9000 g/mol, in particular from 1400 g/mol to 8000 g/mol, in particular from 1500 g/mol to 7000 g/mol, in particular from 1600 g/mol to 6000 g/mol, in particular from 1700 g/mol to 5000 g/mol, in particular from 1800 g/mol to 4000 g/mol, in particular from 1900 g/mol to 3000 g/mol.

In an embodiment, the polyether has a mean molecular weight, determined by size exclusion chromatography, lying in a range of from 7600 g/mol to 9600 g/mol, in particular of from 7650 g/mol to 9550 g/mol, in particular of from 7680 g/mol to 9510 g/mol, in particular of from 7700 g/mol to 9500 g/mol, in particular of from 7800 g/mol to 9400 g/mol, in particular of from 7900 g/mol to 9300 g/mol, in particular of from 8000 g/mol to 9200 g/mol, in particular of from 8100 g/mol to 9100 g/mol, in particular of from 8200 g/mol to 9000 g/mol, in particular of from 8300 g/mol to 8900 g/mol,in particular of from 8400 g/mol to 8800 g/mol, in particular of from 8500 g/mol to 8700 g/mol, in particular of from 8600 g/mol to 8650 g/mol.

In an embodiment, the polyether concentration in the cell-free reaction mixture lies in in a range of from 0.002 % (w/v) to 2.0 % (w/v), in particular from 0.003 % (w/v) to 1.9 % (w/v), in particular from 0.004 % (w/v) to 1.8 % (w/v), in particular from 0.005 % (w/v) to 1.7 % (w/v), in particular from 0.006 % (w/v) to 1.6 % (w/v), in particular from 0.007 % (w/v) to 1.5 % (w/v), in particular from 0.008 % (w/v) to 1.4 % (w/v), in particular from 0.009 % (w/v) to 1.3 % (w/v), in particular from 0.01 % (w/v) to 1.2 % (w/v), in particular from 0.02 % (w/v) to 1.1 % (w/v), in particular from 0.03 % (w/v) to 1.0 % (w/v), in particular from 0.04 % (w/v) to 0.9 % (w/v), in particular from 0.05 % (w/v) to 0.8 % (w/v), in particular from 0.06 % (w/v) to 0.7 % (w/v), in particular from 0.07 % (w/v) to 0.6 % (w/v), in particular from 0.08 % (w/v) to 0.5 % (w/v), in particular from 0.09 % (w/v) to 0.4 % (w/v), in particular from 0.1 % (w/v) to 0.3 % (w/v), in particular from 0.15 % (w/v) to 0.2 % (w/v).

In an embodiment, the power input into the cell-free reaction mixture lies in a range of from 0.5 to 10.0 kW/m³, in particular of from 0.51 to 9.7 kW/m³, in particular of from 1.0 to 9.5 kW/m³, in particular of from 1.5 to 9.2 kW/m³, in particular of from 2.0 to 9.0 kW/m³, in particular of from 2.5 to 8.5 kW/m³, in particular of from 3.0 to 8.0 kW/m³, in particular of from 3.5 to 7.5 kW/m³, in particular of from 4.0 to 7.0 kW/m³, in particular of from 4.5 to 6.5 kW/m³, in particular of from 5.0 to 6.0 kW/m³.

In an embodiment, the power input into the cell-free reaction mixture is at least 0.51 kW/m³, in particular at least 0.6 kW/m³, in particular at least 1.0 kW/m³, in particular at least 2.0 kW/m³.

In an embodiment, the method is carried out at a reaction temperature lying in a range of from 20 °C to 40 °C, in particular of from 25 °C to 37 °C, in particular from 30 °C to 35 °C, in particular from 22 °C to 28 °C, in particular from 24 °C to 26 °C, i.e., at or around 25 °C.

In an embodiment, the cell-free reaction mixture is contained in a well of a microtiter plate. Microtiter plates are generally well known and available in different scales, such as 48-well, 72-well, 96-well, and 384-well plates. Appropriate reaction volumes that can be contained in all wells of a microtiter plate are volumes lying in a range of from 20 µl to 5 ml, in particular from 50 µl to 4.5 ml, in particular from 100 µl to 4 ml, in particular from 200 µl to 3.5 ml, in particular from 300 µl to 3 ml, in particular from 400 µl to 2.5 ml, in particular from 500 µl to 2 ml, in particular from 600 µl to 1.5 ml, in particular from 700 µl to 1 ml. Microtiter plates are particularly easy to handle and can be placed on a microtiter shaking device to achieve an agitation of the reaction mixture contained in the wells of the microtiter plate. The power input into the reaction mixture in a shaken microtiter plate can be calculated according to Dürauer et al. (2016).

In an embodiment, the cell-free reaction mixture is contained in a shake flask. A shake flask has typically a larger volume than a microtiter plate and is thus particularly appropriate for the production of larger amounts of polypeptide. Appropriate reaction volumes that can be contained in a shake flask are volumes lying in a range of from 5 ml to 1000 ml, in particular from 10 ml to 950 ml, in particular from 15 ml to 900 ml, in particular from 20 ml to 850 ml, in particular from 25 ml to 800 ml, in particular from 30 ml to 750 ml, in particular from 35 ml to 700 ml, in particular from 40 ml to 650 ml, in particular from 45 ml to 600 ml, in particular from 50 ml to 550 ml, in particular from 60 ml to 500 ml, in particular from 70 ml to 450 ml, in particular from 80 ml to 400 ml, in particular from 90 ml to 350 ml, in particular from 100 ml to 300 ml, in particular from 125 ml to 250 ml, in particular from 150 ml to 200 ml. The power input into the reaction mixture contained in a shake flask can be calculated according to Büchs et al. (2000).

In an embodiment, the cell-free reaction mixture is contained in a reactor. A reactor has typically a larger volume than a shake flask and is thus particularly appropriate for the production of even larger amounts of polypeptide.

Generally, the presently claimed method is not restricted to a specific type or size of reactor. In an embodiment, however, the reactor is a stirred tank reactor. Appropriate reaction volumes that can be contained in a stirred tank reactor are volumes lying in a range of from 10 ml to 1000 I, in particular from 20 ml to 950 I, in particular from 30 ml to 900 I, in particular from 40 ml to 850 I, in particular from 50 ml to 800 I, in particular from 60 ml to 750 I, in particular from 70 ml to 700 I, in particular from 80 ml to 650 I, in particular from 90 ml to 600 I, in particular from 100 ml to 550 I, in particular from 200 ml to 500 I, in particular from 300 ml to 450 I, in particular from 400 ml to 400 I, in particular from 500 ml to 300 I, in particular from 1 I to 250 I, in particular from 2 I to 200 I, in particular from 5 I to 150 I, in particular from 8 I to 100 I, in particular from 10 I to 75 I, in particular from 15 I to 70 I, in particular from 20 I to 65 I, in particular from 25 I to 60 I, in particular from 30 I to 55 I, in particular from 35 I to 50 I, in particular from 40 I to 45 I. The power input into the reaction mixture contained in a stirred tank reactor can be calculated according to Kaiser et al. (2018).

In an embodiment, the reactor is an orbitally shaken bioreactor, such as the Kuhner Shaker SB3, SB10, SB50, SB200, or SB2000. Appropriate reaction volumes that can be contained in an orbitally shaken bioreactor are volumes lying in a range of from 500 ml to 1000 I, in particular from 1 I to 950 I, in particular from 2 I to 900 I, in particular from 5 I to 850 I, in particular from 10 I to 800 I, in particular from 15 I to 750 I, in particular from 20 I to 700 I, in particular from 25 I to 650 I, in particular from 30 I to 600 I, in particular from 50 I to 550 I, in particular from 60 I to 500 I, in particular from 70 I to 450 I, in particular from 80 I to 400 I, in particular from 90 I to 350 I, in particular from 100 I to 300 I, in particular from 125 I to 250 I, in particular from 150 I to 200 l. The power input into the reaction mixture contained in an orbitally shaken bioreactor can be calculated according to Klöckner et al. (2012).

In an embodiment, the reactor is a rocking motion bioreactor. Appropriate reaction volumes that can be contained in a rocking motion bioreactor are volumes lying in a range of from 100 ml to 500 I, in particular from 120 ml to 95 I, in particular from 150 ml to 90 I, in particular from 200 ml to 85 I, in particular from 500 ml to 80 I, in particular from 1 I to 75 I, in particular from 2 I to 70 I, in particular from 5 I to 65 I, in particular from 10 I to 60 I, in particular from 15 I to 55 I, in particular from 20 I to 50 I, in particular from 25 I to 45 I, in particular from 30 I to 40 I. The power input into the reaction mixture contained in a rocking motion bioreactor can be calculated according to Bai et al. (2019).

A stirred tank bioreactor, an orbitally shaken bioreactor or a rocking motion bioreactor is particularly appropriate to introduce a high power input into the reaction mixture by agitation. A high shaking frequency will result in a high power input and an oxygen transfer rate being sufficient for an efficient cell-free protein synthesis, this being a prerequisite for a high yield of synthesized polypeptide.

In another embodiment, the reactor is a bubble reactor such as a bubble column reactor. A bubble reactor allows the introduction of a gas into the reaction mixture, wherein the ascending and/or bursting bubbles serve for power input into the reaction mixture. In addition, it is possible to introduce a specific gas by the bubbles guided through the reaction mixture in a bubble reactor. Since bursting bubbles often destroy organelles in a cell-free reaction mixture, they are generally connected to certain disadvantages when working in cell-free polypeptide synthesis systems. However, the addition of the polyether in the presently claimed range efficiently prevents the destruction of organelles to a significant extent so that the presently claimed method can be carried out in a bubble reactor. The power input into the reaction mixture contained in a bubble reactor can be calculated according to Sánchez Pérez et al. (2006).

In an embodiment, oxygen is introduced into the cell-free reaction mixture during the cell-free polypeptide synthesis. This can be done, e.g., by introducing air or pure oxygen in gas form into the reaction mixture. If a bubble reactor is used for carrying out the method, the oxygen gas or air can be guided in form of bubbles through the reaction mixture so as to achieve a bubble-aeration of the reaction mixture.

In an embodiment, the cell-free reaction mixture has a volume of at least 300 µl, in particular at least 500 µl, in particular at least 750 µl, in particular at least 1 ml, in particular at least 2 ml, in particular at least 5 ml, in particular at least 10 ml, in particular of at least 20 ml, in particular of at least 50 ml, in particular of at least 100 ml, in particular of at least 500 ml, in particular of at least 1 I, in particular of at least 2 I, in particular of at least 5 ml, in particular of at least 10 I, in particular of at least 20 I. In an embodiment, the reaction mixture has a volume lying in a range of from 300 µl to 100 I, in particular from 500 µl to 90 I, in particular from 1 ml to 80 I, in particular from 5 ml to 70 I, in particular from 10 ml to 60 I, in particular from 20 ml to 50 I, in particular from 50 ml to 40 I, in particular from 100 ml to 30 I, in particular from 200 ml to 20 I, in particular from 300 ml to 10 I, in particular from 500 ml to 5 I, in particular from 1 I to 3 I. While such a reaction mixture volume is very difficult to handle in terms of polypeptide yield without significant agitation of the reaction mixture, it is well possible to produce polypeptides in such a large reaction mixture if the reaction mixture is thoroughly agitated. As explained above, such agitation would lead to a significant (if not even total) decrease of yield if the reaction mixture would not contain the polyether. Thus, the addition of the polyether enables the easy large-scale manufacture of polypeptides.

In an embodiment, the synthesized polypeptide is a protein. Then, the method can also be denoted as method for cell-free protein synthesis.

In an aspect, the present invention relates to the use of a polyether in a concentration lying in a range of from 0.001 % (weight per volume; w/v) to 2 % (w/v) of the cell-free reaction mixture for increasing polypeptide yield in cell-free polypeptide synthesis. In this context, the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol. Since cell-free polypeptide synthesis does not comprise any cells, it is by definition an in vitro method.

In an aspect, the present invention relates to a cell-free reaction mixture for cell-free polypeptide synthesis. This cell-free reaction mixture comprises a nucleic acid having a sequence coding for a polypeptide to be produced. Furthermore, it comprises an RNA polymerase, nucleotides and optionally one or more adjuvants.

What is special about this cell-free reaction mixture is that it further comprises a polyether in a concentration lying in a range of from 0.001 % (w/v) to 2 % (w/v). In this context, the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol. This cell-free reaction mixture is particularly appropriate to synthesize polypeptides in cell-free synthesis systems under agitation resulting in a power input into the cell-free reaction mixture of at least 0.5 kW/m³. While other cell-free reaction mixtures will not provide any significant yields of polypeptides to be synthesized under such conditions, the presently claimed cell-free reaction mixture enables a high yield even under such conditions of high power input into the system.

In an embodiment, the cell-free reaction mixture further comprises chloroplasts, mitochondria and/or plastids.

In an embodiment, the cell-free reaction mixture comprises a cell lysate produced from eukaryotic cells like *Pichia* cells, mammalian cells (like, e.g., CHO cells), insect cells, plant cells (like, e.g., grain germ cells, in particular wheat germ cells) or from prokaryotic cells like bacteria cells (like, e.g., *E. coli* cells). In an embodiment, the cell-free reaction mixture comprises a cell lysate produced from plant cells, such as tobacco (*Nicotiana tabacum*) cells, in particular tobacco BY-2 cells, or maize cells. It turned out that such BY-2 cells are particularly appropriate to be applied in cell-free protein synthesis. For this purpose, BY-2 cells are digested with a cocktail of digestive enzymes that destroy the cell wall but all components that are necessary for protein synthesis remain intact, such as ribosomes and ribosomal enzymes necessary for protein synthesis. Such a cell lysate derived from tobacco BY-2 cells can also be denoted as BY-2 lysate or BYL. It turned out the intrinsic RNA polymerase activity is lost during preparation of the BY-2 lysate. This necessitates subsequent addition of RNA polymerase.

In an embodiment, the nucleic acid having a sequence coding for a polypeptide to be produced is a chemically or biologically synthesized nucleic acid. A chemical synthesis can be performed in a fast and cheap way so that a plurality of different nucleic acids serving as template for an mRNA that will finally be translated into protein can be easily produced according to the users' needs.

In an aspect, the present invention relates to a cell-free reaction mixture comprising a cell lysate and a polyether in a concentration lying in a range from 0.001 % (w/v) to 2 % (w/v). In this context, the cell lysate is produced from prokaryotic cells like bacteria cells or from eukaryotic cells like *Pichia* cells, mammalian cells, insect cells, or plant cells. Furthermore, the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol.

All embodiments of the method can be combined in any desired way and can be transferred either individually or in any arbitrary combination to the use and to any of the cell-free reaction mixtures. Likewise, all embodiments of the use can be combined in any desired way and can be transferred either individually or in any arbitrary combination to the method and to any of the cell-free reaction mixtures. Finally, all embodiments of the cell-free reaction mixtures can be combined in any desired way and can be transferred either individually or in any arbitrary combination to the method, to the use, and to the respective other cell-free reaction mixture.

Further details of aspects of the present invention will be explained in the following making reference to exemplary embodiments and accompanying Figures. In the Figures:
- Figure 1A: shows the relative expression level of enhanced yellow fluorescent protein (eYFP) synthesized in a cell-free protein synthesis system at different power inputs and at a concentration of various antifoam agents of 0.1 % (w/v) (comparative examples);
- Figure 1B: shows the relative expression level of enhanced yellow fluorescent protein (eYFP) synthesized in a cell-free protein synthesis system at different power inputs and at a concentration of various antifoam agents of 1.0 % (w/v) (comparative examples); and
- Figure 2: shows the relative eYFP expression level in a cell-free protein synthesis system at a power input of 0.51 kW/m³, wherein the reaction mixture contained one of various polyethers in various concentrations.

To evaluate the suitability of different antifoam agents in enhancing the yield of cell-free protein synthesis, such a cell-free protein synthesis was performed. For this purpose, a cell-free reaction mixture comprising a plasmid comprising a nucleic acid encoding enhanced yellow fluorescent protein (eYFP) as template for an mRNA production and subsequent protein production has been used. The reaction mixture was filled into the wells of a 48-well microtiter plate. Each well was filled with 300 µl of the reaction mixture. This microtiter plate was agitated by a shaker having a shaking diameter of 3 mm. The protein synthesis was performed at 25 °C.

The shaking frequency was set to 700 rpm, resulting in a power input of approximately 0.19 kW/m³ or, alternatively, to 1000 rpm, resulting in a power input of approximately 0.51 kW/m³.

The power input into the reaction mixture was calculated according to Dürauer et al. (2016).

Afterwards, the amount of eYFP was spectrophotometrically determined via the specific fluorescence of eYFP. The concentration of eYFP was finally determined by a calibration curve based on different standard eYFP samples having a known eYFP concentration. Finally, a relative eYFP expression level was determined, wherein the eYFP expression in a 300-µl reaction mixture without addition of an antifoaming agent shaken at 700 rpm at a shaking diameter of 3 mm under otherwise identical synthesis conditions was set to 100 %.

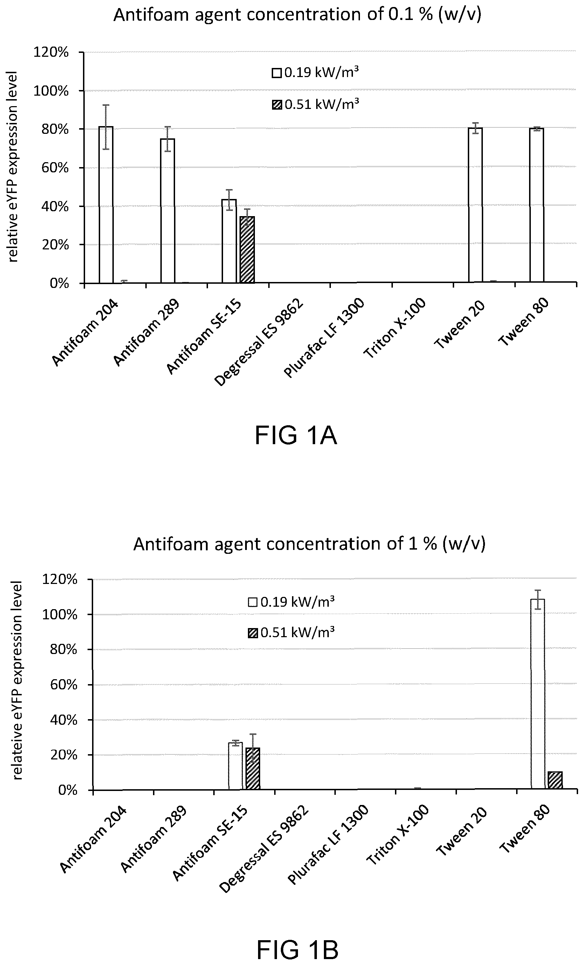

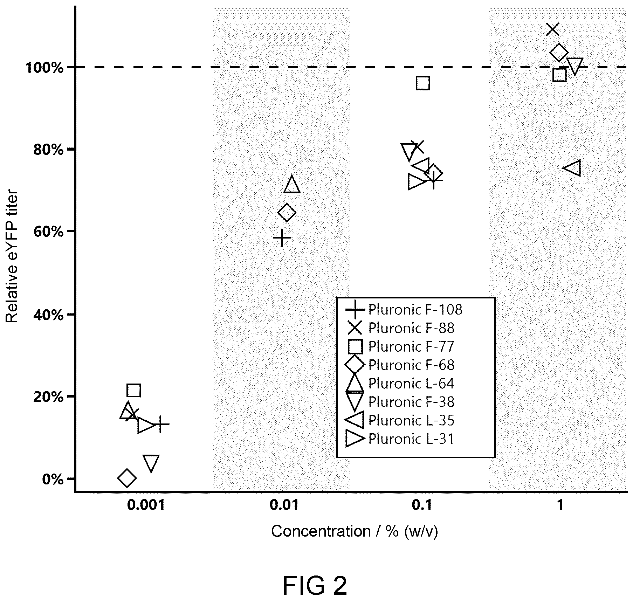

The results of these experiments are depicted in Figures 1A, 1B and 2. In this context, Figure 1A shows comparative examples illustrating the relative eYFP expression levels of cell-free reaction mixtures comprising one of different antifoam agents at a concentration of 0.1 % (w/v) at a power input of approximately 0.19 kW/m³ (non-filled bars) and at a power input of 0.51 kW/m³ (shaded bars). While at the lower power input, five of the tested antifoam agents were able to stabilize the reaction mixture such that at least 40 % of the standard eYFP expression level could be reached, only a single antifoam agent, namely Antifoam SE-15, was able to show this effect also with the higher power input into the reaction mixture. None of the other tested antifoam agents was able to stabilize the reaction mixture sufficiently, as a result of which no protein expression at all could be detected at the higher power input. The addition of three of the tested antifoam agents did not result in any protein expression, neither at agitation of 700 rpm resulting in the lower power input nor at agitation of 1000 rpm resulting in the higher power input.

When increasing the antifoam agent concentration in the cell-free reaction mixture to 1 %, only two antifoam agents were suited to stabilize the reaction mixture such that protein expression was still possible. The corresponding results are depicted as further comparative examples in Figure 1B. The addition of Tween 80 resulted in an eYFP expression that was higher than 50 % of the standard eYFP expression when the microtiter plate was agitated with a power input of approximately 0.19 kW/m³. Upon increasing the power input to 0.51 kW/m³, the relative protein expression in case of adding Tween 80 dropped to approximately 10 % of the standard eYFP expression. Antifoam SE-15 showed similar eYFP expression levels at a power input of 0.19 kW/m³ and 0.51 kW/m³. However, the eYFP expression level remained at approximately 25 %, i.e., at a comparatively low level.

The situation is totally different when adding a polyether having specific characteristics to the reaction mixture in different concentrations. The results of the according experiments performed at a shaking frequency of 1000 rpm resulting in a high power input of approximately 0.51 kW/m³ are shown in Figure 2. The tested polyethers were poloxamer 188 (Pluronic F-68), Pluronic F-88, Pluronic F-77, Pluronic F-38, Pluronic F-108, Pluronic L-64, Pluronic L-35, and Pluronic L-31. These substances fulfil the requirements of claim 1 with respect to the polyether to be used in the claimed method.

Already at a concentration of as low as 0.001 % (w/v), a yield-enhancing effect could be observed for basically all tested polyethers. It should be noted that no eYFP expression at all could be observed when agitating the microtiter plate with 1000 rpm leading to a power input of approximately 0.51 kW/m³ into the reaction mixture if no polyether as yield-enhancing substance was added.

Upon increasing the polyether concentration to 0.01 % (w/v) or higher, the relative eYFP titer increased to values between 60 % and more than 100 %. Thus, the tested polyethers are - in contrast to the other tested antifoaming substances - highly appropriate for enhancing the yield of eYFP production under high power input into the reaction mixture.

This data clearly indicates that the tested polyethers have - in contrast to all other tested antifoam agents - an unexpected and surprising effect on the protein expression yield in a cell-free protein synthesis system that is agitated with a power input of more than 0.19 kW/m³, in particular of 0.5 kW/m³ (or 0.51 kW/m³, respectively) or more.

### List of non-patent references cited in the preceding sections

1. Bai, Y., Moo-Young, M., & Anderson, W. A. (2019). Characterization of power input and its impact on mass transfer in a rocking disposable bioreactor. Chemical Engineering Science, 209, 115183.
2. Büchs, J., Maier, U., Milbradt, C., & Zoels, B. (2000). Power consumption in shaking flasks on rotary shaking machines: I. Power consumption measurement in unbaffied flasks at low liquid viscosity. Biotechnology and Bioengineering, 68(6), 589-593.
3. Dürauer, A., Hobiger, S., Walther, C., & Jungbauer, A. (2016). Mixing at the microscale: Power input in shaken microtiter plates. Biotechnology Journal, 11(12), 1539-1549.
4. Gigout, A., Buschmann, M. D., & Jolicoeur, M. (2008). The fate of Pluronic F-68 in chondrocytes and CHO cells. Biotechnology and Bioengineering, 100(5), 975-987.
5. Kaiser, S. C., Werner, S., Jossen, V., Blaschczok, K., & Eibl, D. (2018). Power input measurements in stirred bioreactors at laboratory scale. JoVE (Journal of Visualized Experiments), (135), e56078.
6. Klöckner, W., Tissot, S., Wurm, F., & Büchs, J. (2012). Power input correlation to characterize the hydrodynamics of cylindrical orbitally shaken bioreactors. Biochemical Engineering Journal, 65, 63-69.
7. Sánchez Pérez, J. A., Porcel, E. R., López, J. C., Sevilla, J. F., & Chisti, Y. (2006). Shear rate in stirred tank and bubble column bioreactors. Chemical Engineering Journal, 124(1-3), 1-5.
8. Tharmaiingam, T., Ghebeh, H., Wuerz, T., & Butler, M. (2008). Pluronic enhances the robustness and reduces the cell attachment of mammalian cells. Molecular Biotechnology, 39, 167-177.

## Claims

1. Method for cell-free polypeptide synthesis, comprising translating mRNA in a cell-free reaction mixture under agitation of the cell-free reaction mixture,
**characterized**
**in that** the agitation is performed with a power input into the cell-free reaction mixture of at least 0.5 kW/m³ and in that the cell-free reaction mixture comprises a polyether in a concentration lying in a range from 0.001 % (w/v) to 2 % (w/v), wherein the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol.

2. Method according to claim 1, **characterized in that** the polyether fulfils one of the following criteria:
i) the weight percentage of the hydrophilic units lies in a range of from 80 % to 100 % and the molecular weight of the polyether lies in a range of from 200 g/mol to 20000 g/mol;
ii) the weight percentage of the hydrophilic units lies in a range of from 30 % to 79.9 % and the molecular weight of the hydrophobic units lies in a range of from 200 g/mol to 3500 g/mol;
iii) the weight percentage of the hydrophilic units lies in a range of from 10 % to 29.9 % and the molecular weight of the hydrophobic units lies in a range of from 200 g/mol to 1500 g/mol;

3. Method according to claim 1 or 2, **characterized in that** the polyether comprises at least one hydrophilic block of hydrophilic units and at least one hydrophobic block of hydrophobic units.

4. Method according to claim 3, **characterized in that** the polyether comprises one central hydrophobic block and two lateral hydrophilic blocks, wherein each hydrophilic block is covalently bound to a different terminus of the hydrophobic block.

5. Method according to any of the preceding claims, **characterized in that** the polyether is a polyethylene glycol or a poloxamer.

6. Method according to claim 5, **characterized in that** the poloxamer is chosen from the group consisting of poloxamer 188, Pluronic F-88, Pluronic F-77, Pluronic F-38, Pluronic F-108, Pluronic L-64, Pluronic L-35, and Pluronic L-31.

7. Method according to any of the preceding claims, **characterized in that** the power input into the cell-free reaction mixture lies in a range of from 0.5 kW/m³ to 10.0 kW/m³.

8. Method according to any of the preceding claims, **characterized in that** the cell-free reaction mixture is contained in a well of a microtiter plate, in a shake flask, or in a reactor.

9. Method according to any of the preceding claims, **characterized in that** the cell-free reaction mixture has a volume of at least 5 ml.

10. Use of a polyether in a concentration lying in a range from 0.001 % (w/v) to 2 % (w/v) of a cell-free reaction mixture for increasing polypeptide yield in cell-free polypeptide synthesis, wherein the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol.

11. Cell-free reaction mixture for cell-free polypeptide synthesis, comprising a nucleic acid having a sequence coding for a polypeptide to be produced, an RNA polymerase, nucleotides and optionally one or more adjuvants,
**characterized**
**in that** the cell-free reaction mixture further comprises a polyether in a concentration lying in a range from 0.001 % (w/v) to 2 % (w/v), wherein the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol.

12. Cell-free reaction mixture according to claim 11, **characterized in that** the cell-free reaction mixture further comprises chloroplasts, mitochondria and/or plastids.

13. Cell-free reaction mixture according to claim 11 or 12, **characterized in that** the cell-free reaction mixture comprises a cell lysate produced from prokaryotic cells like bacteria cells or from eukaryotic cells like *Pichia* cells, mammalian cells, insect cells, or plant cells.

14. Cell-free reaction mixture according to any of claims 11 to 13, **characterized in that** the nucleic acid having a sequence coding for a polypeptide to be produced is chemically synthesized.

15. Cell-free reaction mixture comprising a cell lysate and a polyether in a concentration lying in a range from 0.001 % (w/v) to 2 % (w/v), wherein the cell lysate is produced from prokaryotic cells like bacteria cells or from eukaryotic cells like *Pichia* cells, mammalian cells, insect cells, or plant cells, and wherein the polyether comprises hydrophilic units and optionally hydrophobic units, wherein a weight percentage of the hydrophilic units lies in a range of from 10 % to 100 % and wherein the molecular weight of the polyether lies in a range of from 200 g/mol to 20000 g/mol.
